# EUROPEAN PATENT APPLICATION

(11) **EP 2 682 048 A1**
(43) Date of publication of application: **08.01.2014**
(21) Application number: 12752571.5
(22) Date of filing: 27.02.2012
(51) Int. Cl.: A61B 3/14

(54) **OPHTHALMOLOGIC IMAGING APPARATUS**

(30) Priority: 02.03.2011 JP 2011045037
(71) Applicant: Kowa Company Ltd., Naka-ku Nagoya-shi Aichi 460-8625 (JP)
(72) Inventor: MIZUOCHI, Masaharu, Shizuoka 431-2103 (JP)
(74) Representative: Kronthaler, Wolfgang N.K.
(86) International application number: PCT/JP2012/054767
(87) International publication number: WO 2012/118010

(57) **Abstract**

Provided are an illumination optical system (100, 13, 15...) for projecting illumination light from an illumination light source onto a fundus of a subject eye; a photography optical system (22, 42, 31, 32...) for taking an image of the fundus illuminated by the illumination light; a first electronic infrared imaging means (40) for taking a video for observing the fundus of the subject eye during the subject eye observation period; an exciter filter (13) for photographing natural fluorescence from the fundus of the subject eye and a barrier filter (BF3) for photographing natural fluorescence from the fundus of the subject eye respectively capable of being inserted into and retracted from optical paths of the illumination optical system and the photography optical system after the subject eye observation period in which the fundus of the subject eye is observed using the first electronic imaging means and prior to taking a still image of the subject eye by a second electronic imaging means (53); and an adjustment means for varying the ratio of the visible light component to the infrared light component in the illumination light.

## Description

### Technical Field

The present invention relates to an ophthalmologic photography apparatus for performing natural fluorescence photography such as fundus autofluorescence (FAF) photography.

### Background Art

Fundus photography with fundus autofluorescence (FAF) has been the subject of attention in recent years. Fundus autofluorescence (FAF) photography is a fundus photography technique utilizing the natural fluorescence of the fundus.

In age-related macular degeneration, for example, lipofuscin accumulates in the macular area of the fundus retina. The accumulated lipofuscin is a type of fluorescent substance, and emits natural fluorescence when irradiated with light of a specific wavelength. Not only for lipofuscin in age-related macular degeneration, but also for fluorescent substances unique to other lesions and conditions, it is possible to perform fundus autofluorescence (FAF) photography using a similar photography method by appropriately selecting the properties of an exciter filter and a barrier filter that are inserted in the observation/photography optical system.

FAF emits extremely faint levels of fluorescence, and photographed images thereof contain high levels of noise, necessitating high levels of light radiation during photography or a high-sensitivity imaging device. Regarding image noise in particular, in order to achieve noise reduction effects, a configuration is proposed (for example, Patent Document 1) in which proper exposure is set while avoiding pupillary contraction when synthesizing multiple photographed fundus images.

### Prior Art Documents

### Patent Documents

Patent Document 1: Japanese Laid-open Patent Application Publication No. 2010-259531

### Summary of Invention

### Problems to be Solved

FAF photography is a method of photographing the autofluorescence of the fundus using a visible light (green light) exciter filter and a near-infrared light (red light) fluorescence filter. Typically, the following two photographic methods are used.

The first method employs a non-mydriatic fundus camera arrangement in which infrared light is used for observation with a light-receiving element such as a CCD camera and display means such as an LCD monitor, and photographs are taken via an exciter filter. The other method employs a mydriatic fundus camera arrangement in which visible light is used to perform direct observation via a finder, and a fluorescence filter (barrier filter) is inserted when photographs are taken.

FAF photography itself is a noninvasive method of fluorescence photography allowing images to be taken without the use of drugs (for example, without the need for intravenous injection of any fluorescent agent). However, the mydriatic photography method described above uses mydriatic agents, placing a great burden upon the subject. In non-mydriatic photography, meanwhile, a problem arises in that the FAF image is darkened due to the lack of visual pigment bleaching.

By the way, visual pigment bleaching is said to enhance fundus autofluorescence (FAF). It is therefore advantageous to irradiate the fundus with visible light prior to photography, thereby inducing visual pigment bleaching, in which state fundus photography can be performed to obtain a bright FAF image having enhanced autofluorescence. However, when photographing a subject eye using a non-mydriatic photography method, irradiation of the subject eye with visible light prior to photography typically induces pupillary contraction in the subject eye, impeding non-mydriatic photography. There is therefore a need to perform visible light irradiation at a weak, non-pupillary contraction-inducing level, promoting visual pigment bleaching without inducing pupillary contraction.

An object of the present invention is that, especially in a non-mydriatic photography method, a weak level of visible light is projected to promote visual pigment bleaching in order to obtain a brighter fundus image in natural fluorescence photography such as fundus autofluorescence (FAF) photography without the need for complicated image processing involving photographing and synthesizing a plurality of fundus images.

### Means for Solving the Problems

In order to achieve the object described above, the present invention provides an ophthalmologic photography apparatus that comprises an illumination optical system for illuminating the fundus of a subject eye using illumination light from an illumination light source; a photography optical system for taking a fundus image of the fundus irradiated with the illumination light by the illumination optical system; a first electronic infrared imaging means for taking a video for observing the fundus of the subject eye during a subject eye observation period prior to taking a still image of the subject eye; and a second electronic imaging means for taking a still image of the subject eye. In the apparatus, the subject eye observation period in which the fundus of the subject eye is observed using the first electronic imaging means is followed by taking a still image of the subject eye using the second imaging means in response to a shutter operation and recording the obtained still image as still image information. The ophthalmologic photography apparatus is provided with an exciter filter removably provided in an optical path of the illumination optical system for photographing natural fluorescence emitted by the fundus of the subject eye; a barrier filter removably provided in an optical path of the photography optical system for photographing natural fluorescence emitted by the fundus of the subject eye; and an adjustment means for varying the ratio of the visible light component to the infrared light component of the illumination light.

### Effect of the Invention

In accordance with the configuration described above, in natural fluorescence photography such as fundus autofluorescence (FAF) photography in which the exciter filter and barrier filter are inserted into their respective optical paths, it is possible to vary the ratio of the visible light component to the infrared light component of the illumination light source using the adjustment means during the subject eye observation period in which the fundus of the subject eye is observed using the first electronic infrared imaging means and irradiate the fundus of the subject eye with a weak level of visible light promoting bleaching of the visual pigment of the fundus without inducing subject eye pupillary contraction that would impede subsequent natural fluorescence photography. Particularly in the non-mydriatic photography method, a weak level of visible light can promote bleaching of the visual pigment of the fundus and provide a brighter fundus image in natural fluorescence photography such as fundus autofluorescence (FAF) photography.

### Brief Description of Drawings

FIG. 1 is an illustrative view showing the configuration of a mydriatic/non-mydriatic integrated ophthalmologic photography apparatus employing the present invention;
FIG. 2 is an illustrative view showing the transmission properties of an exciter filter and a barrier filter used in fundus autofluorescence (FAF) photography;
FIG. 3 is an illustrative view showing the configuration of a non-mydriatic ophthalmologic photography apparatus employing the present invention;
FIG. 4 is an illustrative view showing the detailed configuration of an illumination system of infrared light LED and visible light LED in the ophthalmologic photography apparatus shown in FIG. 3;
FIG. 5 is an illustrative view showing a configuration including switching between exciter filters having different transmittance for visible light and infrared light; and
FIG. 6 is a flow chart showing a fundus autofluorescence (FAF) photography procedure using the ophthalmologic photography apparatus employing the present invention.

### Mode of Carrying Out the Invention

The present invention will be described in detail hereafter with reference to the embodiments shown in the drawings.

### Embodiments

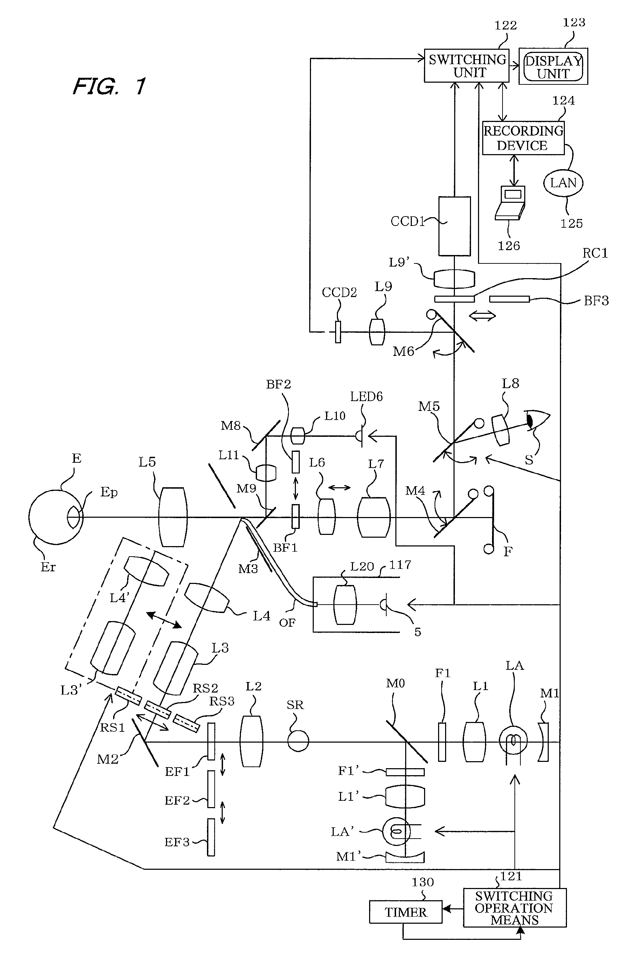

FIG. 1 shows a fundus camera as an embodiment of the ophthalmologic photography apparatus employing the present invention capable of photographing the fundus in a non-mydriatic photography mode and a mydriatic photography mode.

The fundus camera shown in FIG. 1 performs fluorescein angiography in mydriatic photography mode, as well as fundus autofluorescence (FAF) photography (natural fluorescence photography), infrared observation/photography, and indocyanine green angiography (ICG) in non-mydriatic photography mode.

The lower part of FIG. 1 illustrates the configuration of an illumination optical system for projecting illumination light from an illumination light source (lamps LA, LA' ) onto the fundus of a subject eye. Disposed in the lower right part of FIG. 1 are a light source unit for generating visible illumination light including excitation light and a light source unit for infrared light.

Both of the light source units use lamps (such as halogen lamps) LA, LA' as sources of infrared light and visible light, the lamps LA, LA' being disposed at the centers of curvature of spherical mirrors M1 and M1' .

Condenser lenses L1, L1' are disposed in front of the lamps LA, LA' . A filter F1 having infrared-cutting/visible light-passing properties is disposed in front of the condenser lens L1, and a filter F1' having infrared-passing/visible light-cutting properties is disposed in front of the condenser lens L1' .

Of the two filters, the filter F1 transmits only visible illumination light in the direction of the subject eye, cutting the infrared spectrum from roughly 800 nm and up and transmitting the visible spectrum at wavelengths below it. The filter F1' , conversely, cuts the visible spectrum from roughly 800 nm and down and transmits the infrared spectrum at wavelengths above it.

Illumination light from the two light source units is projected through a mirror M0 having infrared-reflecting/visible light-passing properties. The illumination light passes through a condenser lens L2, is reflected by a reflection mirror M2, passes through relay lenses L3, L4 and is reflected by a perforated reflection mirror M3. The light then passes through an objective L5 and impinges on the fundus Er through the pupil Ep of a subject eye E.

A switching operation means 121 described below as well as a switching unit 122 can turn on or off the two light source unit lamps LA, LA' and control the amount of light thereof, constituting adjustment means for varying the ratio of the visible light component to the infrared light component of the illumination light sources.

Exciter filters EF1, EF2, EF3 are disposed at positions between the condenser lens L2 and the reflection mirror M2 so that either one of them can be used and inserted.

The exciter filter EF1 is used for fluorescein angiography, the exciter filter EF2 for fundus autofluorescence (FAF) photography, and the exciter filter EF3 for indocyanine green angiography (ICG).

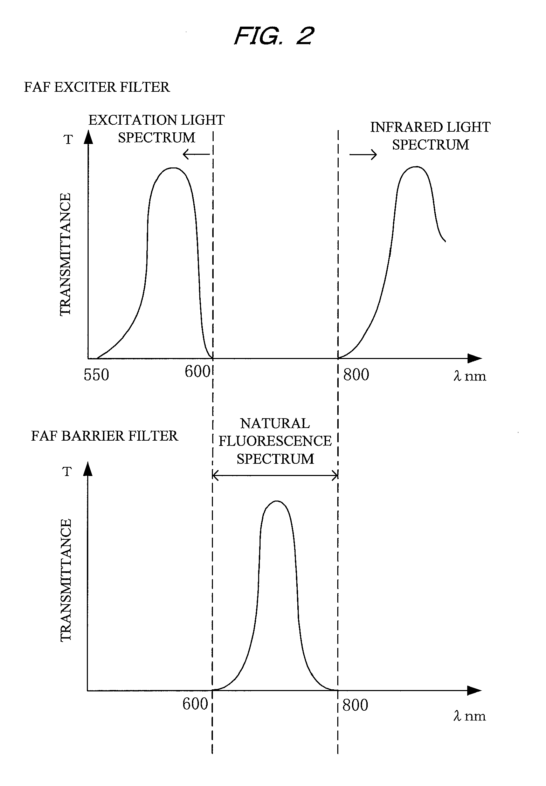

The exciter filter EF2, which is used particularly in fundus autofluorescence (FAF) photography, has two transmission regions, one for excitation light in the visible spectrum (for example, near 550-600 nm) and one for infrared light (for example, 800 nm and up), as shown in the upper part of FIG. 2.

Ring slits RS1, RS2, RS3 for use respectively in non-mydriatic photography mode, mydriatic photography mode and indocyanine green angiography mode are disposed in front of the reflection mirror M2 so that one of them can be inserted.

A strobe light SR serving as a photography light source is disposed between the mirror M0 and the condenser lens L2 for use when photographing a fundus image on a film F or cameras CCD1, CCD2 described hereafter. The strobe light SR has light-emitting properties centered on the excitation light-including visible spectrum.

The relay lenses L3, L4 described above are for use in non-mydriatic mode, and changed to relay lenses L3' , L4' in mydriatic mode and fluorescent mode. A black-spot plate (not shown) or the like may also be disposed near the relay lenses L3, L4 as necessary in order to remove harmful light resulting from reflection at the interface of the objective L5.

The switching operation means 121, comprising switches and necessary drive circuits, selects the relay lenses of the non-mydriatic illumination system (L3, L4) and the mydriatic illumination system (L3' , L4' ), also controlling the switching unit 122 for selecting the outputs from CCD2 and CCD1 described blow. The switching operation means 121 also controls the insertion and removal of the exciter filters EF1-EF3 necessary for fluorescent mode and barrier filters BF1-BF3 described below, switching of return mirrors M4, M5, M6, and turning-on and-off of the observation light sources LA, LA' . A timer (time measurement means) 130 is connected to the switching operation means 121, which turns the timer on and off during fluorescein angiography and indocyanine green angiography.

Light reflected from the fundus Er is received back through the pupil Ep and the objective L5, passes through the perforated reflection mirror M3, a focus lens L6, and an imaging lens L7, and is made incident on the mirror M4. Light reflected from the mirror M4 reflects off the mirror M5 and is observed by an examiner S via an eyepiece L8.

A barrier filter BF1 for fluorescein angiography and a barrier filter BF2 for indocyanine green angiography are disposed so that one of them can be inserted in front of the focus lens L6.

A film F is disposed behind the mirror M4, and, when a fundus image is being photographed on the film F, the mirror M4 is removed from the optical path, directing the fundus image onto the film F.

The mirror M5 is configured so as to be removable from the optical path, and, when the mirror M5 is removed from the optical path, the light beam reflected by the mirror M4 reflects off the mirror M6, passes through a lens L9, and forms an image on a CCD2 for observing an infrared light fundus image.

The CCD2 constitutes a first infrared electronic imaging means that takes a video for the purpose of observing the fundus of the subject eye during an eye observation period prior to taking a still image of the subject eye.

The mirror M6 is a return mirror (or a half mirror) and is configured so that the image of the subject eye passes through a lens L9' and is formed on a CCD1 for capturing a visible light image. The CCD1 constitutes a second electronic imaging means for taking a still image of the subject eye, and, in the present embodiment in particular, has a sensitivity range in the visible and infrared spectra in order to photograph the natural fluorescence of the fundus.

An infrared-cutting filter RC1 or a barrier filter BF3 used for fundus autofluorescence (FAF) photography can be inserted in front of the lens L9' .

The properties of the FAF photography barrier filter BF3 are such that it transmits the fundus autofluorescence (natural fluorescence) spectrum near 600-800 nm, as shown in the lower part of FIG. 2.

The CCD2 is used for infrared light observation (or photography), for simultaneous infrared and visible light observation (or photography), or fluorescent image observation (or photography). Meanwhile, the CCD1 is used for visible light observation or FAF photography, and is a CCD for color photography (either single- or three-plate CCD) having specific sensitivities to the wavelengths of the three primary colors R, G, B. The switching unit 122 controlled by the switching operation means 121 selects the output of either the CCD1 or the CCD2 for supply to a subsequent circuit.

An image filing system or display device can be connected after the switching unit 122. In the embodiment shown in FIG. 1, a display unit 123 and a recording device 124 are connected to the switching unit 122. The display unit 123 can be constituted by a CRT display or an LCD display, and is capable of displaying an image of the subject eye captured by the CCD1 or the CCD2 or various types of associated data. The recording device 124 can be constituted by any desired external storage device such as an HDD, CDR, DVD-RAM, or MO, and stores photographed images of the subject eye or various types of associated data. In FIG. 1, a personal computer (PC) 126 is shown as an example of means for controlling the data filing of the recording device 124, but the recording device 124 may also be constituted by an external storage device built into the PC 126. The data stored in the recording device 124 can be shared (sent/received) with another examination device or PC via a LAN 125.

An LED 6 is provided as a focus dot (FD) light source, and light from the LED 6 passes through a lens L10, a mirror M8 and a lens L11, impinges on a mirror M9 disposed between the perforated reflection mirror M3 and the imaging lens L6, and passes through the aperture in the perforated reflection mirror M3 and the objective L5 to form a focusing spot image on the fundus. The LED 6 is a light-emitting diode for emitting near-infrared light centered on a wavelength of roughly 660 nm.

The fundus camera according to the present embodiment is further provided with a working dot light source 117 for projecting a target for aligning the subject eye E and the fundus camera. In the present embodiment, an optical fiber OF has at one end an end surface, which is disposed on the perforated reflection mirror M3 and forms an optical image of a working dot (WD). The working dot light source 117 is constituted by a lens L20 and an LED 5.

The optical image of the working dot (WD) on the end surface of the optical fiber OF is projected through the objective L5 onto the cornea of the subject eye E. The end surface of the optical fiber OF is disposed at a position such that it comes into focus when the working distance between the subject and the fundus camera reaches a proper distance. The LED 5, like the LED 6, is a light-emitting diode for emitting near-infrared light centered on a wavelength of roughly 660 nm.

In the configuration described above, the switching operation means 121 controls the two light source units for infrared light and visible light according to whether the apparatus is operating in non-mydriatic mode or mydriatic mode, and selects the relay lenses L3, L4 or L3' , L4' .

Of the various types of fundus fluorescence photography, fluorescein angiography and indocyanine green angiography (ICG) are performed using various members in a similar manner to the prior art. These types of fundus fluorescence photography are performed by injecting the subject with a fluorescent agent, and, according to the time marked by the timer 130, the switching operation means 121 selects the exciter filter EF1 for fluorescein angiography, exciter filter EF3 for indocyanine green angiography (ICG), barrier filter BF1 for fluorescein angiography, and barrier filter BF2 for indocyanine green angiography.

In the configuration shown in FIG. 1, fundus autofluorescence (FAF) photography is performed using the natural fluorescence of the fundus without injecting a fluorescent agent.

In fundus autofluorescence (FAF) photography mode which is performed using non-mydriatic photography, an infrared light source of the lamp LA' , condenser lens L1' and filter F1' is used during the eye observation period in which an infrared electronic imaging means (CCD2) is used to observe the fundus of the subject eye. In the present embodiment, the visible light source constituted by the lamp LA, condenser lens L1 and filter F1 is also simultaneously turned on to a weak level of light by the switching operation means 121 after alignment of the anterior ocular segment has been attained.

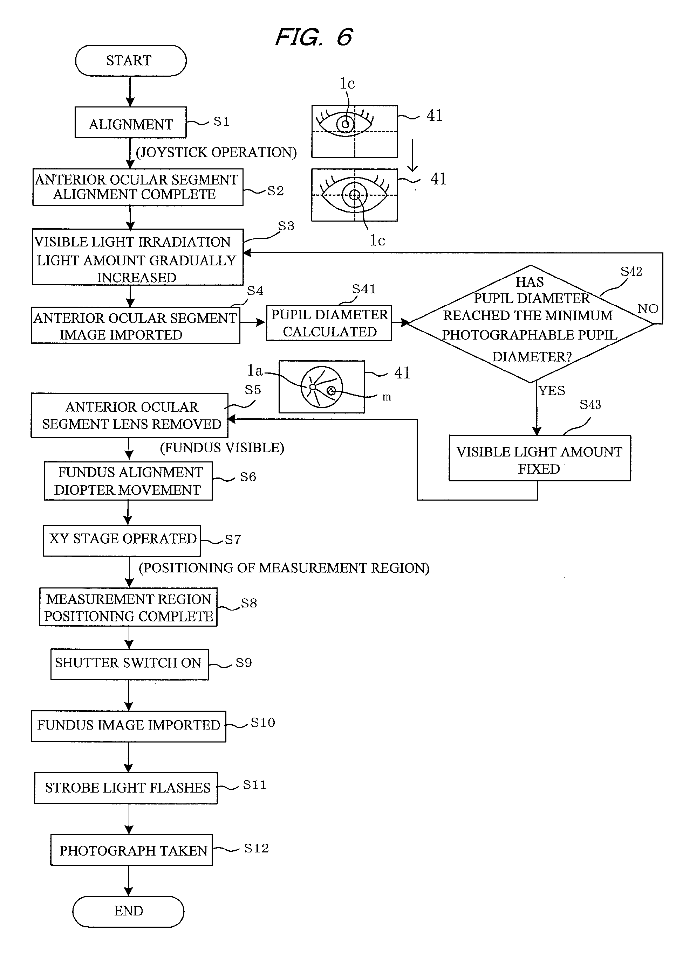

As described in detail, for example, in FIG. 6, prior to the fundus autofluorescence (FAF) photography performed using the CCD1 (or a film F1), the exciter filter EF2 and barrier filter BF3 for fundus autofluorescence (FAF) photography are inserted into the optical path. The visible light source constituted by the lamp LA and condenser lens L1 is operated to irradiate the fundus of the subject eye E with a weak level of visible light with its amount of light being controlled so to be gradually increased. When the pupil diameter of the subject eye E reaches a predetermined size, for example, a minimum photographable diameter, the amount of visible light emitted by the visible light source constituted by the lamp LA, condenser lens L1, and filter F1 is fixed.

Subsequently, fundus autofluorescence (FAF) photography is performed after fundus alignment.

In this way, prior to fundus autofluorescence (FAF) photography, the fundus of the subject eye E is irradiated with a weak level of visible light and the amount of light is raised to and fixed at a maximum within a range not impeding photography. This promotes bleaching of the visual pigment of the fundus, enhancing fundus autofluorescence, and allowing a bright FAF image to be photographed.

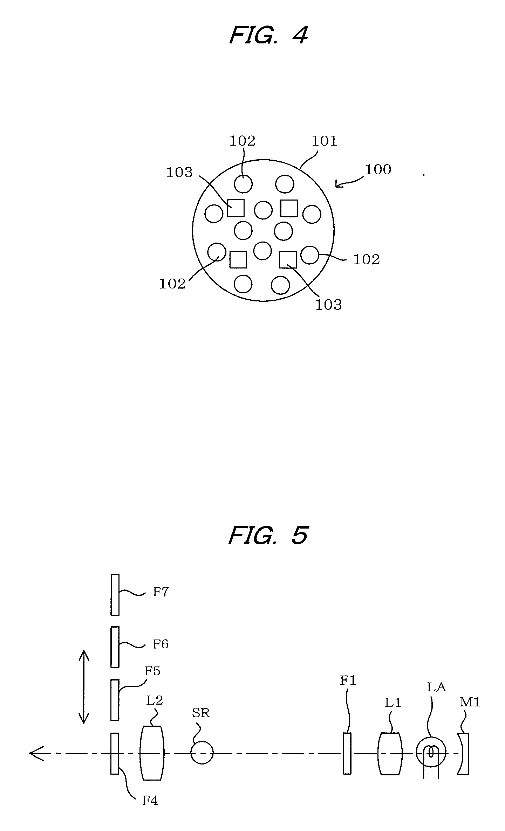

In the fundus autofluorescence (FAF) photography, the natural fluorescence-emitting substance has been assumed to be lipofuscin. However, for photography using natural fluorescence from another fluorescent substance different from lipofuscin and maybe present in the fundus, filters F4, F5, F6, F7... that transmit light of excitation light wavelengths for different fluorescent substances and infrared light wavelengths are prepared, as shown in Fig. 5, in place of the filter EF2 in FIG. 1 according to the excitation light and fluorescence wavelengths. A proper one of the filters F4, F5, F6, F7 can be selected and used according to the fluorescent substance to be measured. Similarly, for the barrier filter BF3, different barrier filters are prepared that transmits light of wavelengths according to the excitation light and fluorescence wavelengths in accordance with the fluorescent substance to be measured, and one of the filters can be selected and used that matches the fluorescent substance to be measured.

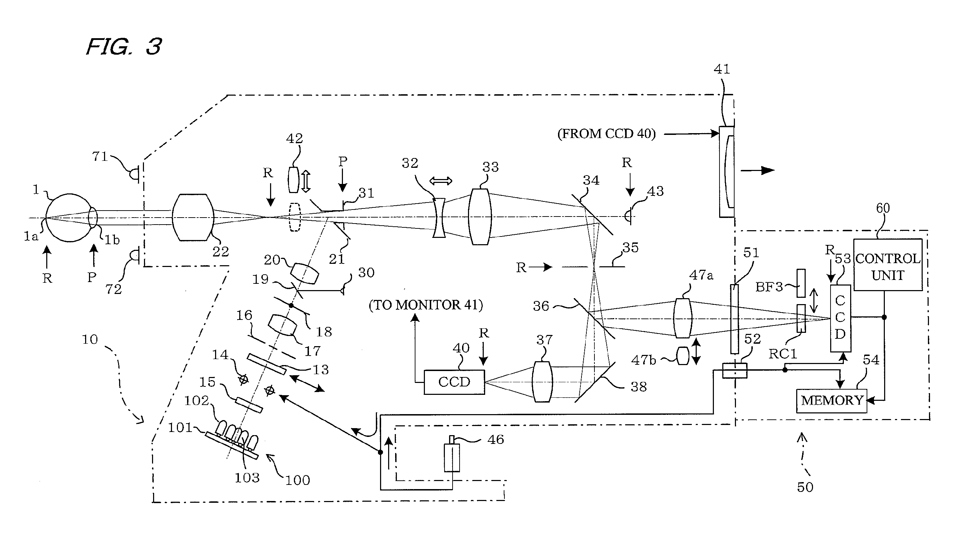

In the above-described embodiment, fundus autofluorescence (FAF) photography is performed by a mydriatic/non-mydriatic integrated ophthalmologic photography apparatus, but similar fundus autofluorescence (FAF) photography can also be performed using the fundus camera capable of non-mydriatic fundus photography as shown in FIG. 3.

The ophthalmologic photography apparatus in FIG. 3 is constituted by a non-mydriatic fundus camera. In the fundus camera shown in FIG. 3, a main unit 10 is provided with an illumination optical system for illuminating the fundus and an image-forming optical system for forming an image of the illuminated fundus. The main unit 10 is disposed on an XY stage not shown in the drawing, and is configured so as to be capable of being positioned (aligned) with respect to a subject eye 1.

In the illumination optical system, light emitted from a light source unit 100 constituted by an LED passes through a diffuser plate 15 and strobe light 14, impinges on a removably disposed exciter filter 13, and illuminates a ring slit 16 disposed at a position conjugate with an anterior ocular segment (pupil) 1b of the subject eye 1.

The exciter filter 13, like the exciter filter EF2 described above, is used for fundus autofluorescence (FAF) photography, and has the properties shown in the upper part of FIG. 2.

The light source unit 100 is constituted by a plurality of semiconductor light-emitting elements (LEDs) arrayed in rows on a board 101.

Disposed are, as shown in FIG. 4, a plurality of infrared light-emitting LEDs 102 and visible light-emitting LEDs 103. In this configuration, the subject eye 1 is irradiated with illumination light comprising a weak visible light component and an infrared light component during the subject eye observation period, and it is possible to vary the ratio of the visible light component to the infrared light component of the illumination light.

Moreover, LEDs 102, 103 provided in the light source unit 100 can be turned on or off or respective light amounts thereof can be controlled. Therefore, the filters (F1, F1' ) that transmit/block visible light and infrared light are obviated, although they are necessary for the illumination system shown in FIG. 1.

The light source unit 100 so configured is not only advantageous in terms of heat generation and noise as compared with a lamp light source such as a halogen lamp as shown in FIG. 1, but also it is possible to individually adjust the amounts of infrared light and visible light, allowing a variety of illumination properties to be easily obtained. This configuration can also be applied to the mydriatic/non-mydriatic integrated ophthalmologic photography apparatus shown in FIG. 1. In this case, the infrared illumination light and the visible excitation light in indocyanine green angiography (ICG) mode and fundus autofluorescence (FAF) photography mode can be easily controlled in terms of the wavelengths and intensity, allowing a high degree of freedom in control.

In FIG. 4, the visible light-emitting LEDs 103 are shown having rectangular shapes, but this is simply a graphical means for distinguishing from the infrared light-emitting LEDs 102 (shown as circles), and does not necessarily represent the actual shapes of the LEDs.

The illumination light that has passed through the ring slit 16, passes through a lens 17, a black-spot plate 18 for removing reflection from an objective 22, a half mirror 19 and a relay lens 20, and reflects off a perforated reflection mirror 21 in the center of which an aperture is formed. The light then passes through the objective 22, and is made incident on a fundus 1a through an anterior ocular segment 1b of the subject eye 1 to illuminate the fundus 1a thereof.

The light reflected from the fundus 1a is received through the objective 22, passes through the aperture in the perforated reflection mirror 21, a photography aperture 31, a focus lens 32 and an imaging lens 33, reflects off a half mirror 34, and passes through a field stop 35 disposed at a position conjugate with the fundus 1a, then impinging on an infrared-passing visible light-reflecting mirror 36.

The infrared light that has passed through the infrared-passing visible light-reflecting mirror 36, reflects off a mirror 38, passes through an imaging lens 37, and impinges on an imaging device 40 constituted by an infrared-sensitive infrared CCD. An output signal from the imaging device 40 is inputted to and displayed on a monitor 41.

The imaging device 40 constitutes an infrared electronic imaging means for recording a video for the purpose of observing the fundus of the subject eye during a subject eye observation period prior to taking a still image of the subject eye.

The visible light reflected by the mirror 36 passes through one of at least two magnification lenses 47a, 47b, is made incident on an attachment unit 50, and is received by an imaging device 53 constituted by a visible light-sensitive visible light CCD.

The imaging device 53 constitutes a visible light electronic imaging means for taking a still image of the subject eye during fundus autofluorescence (FAF) photography mode, and has a visible light and infrared light sensitivity range similar to that of the CCD1 used to photograph the natural fluorescence of the fundus.

An infrared-cutting filter RC1 for non-mydriatic visible light photography or a barrier filter BF3 for fundus autofluorescence (FAF) photography is disposed in the optical path in front of the imaging device 53, and can be switched and used according to the photography mode. The properties of the barrier filter BF3 for FAF photography are similar to those shown, for example, in the lower part of FIG. 2.

The attachment unit 50 is detachably attached to a mount 51 fixed near a pupil-conjugate position on the main unit 10, and, when mounted thereon, receives a shutter operation signal from a shutter 46 via a connector 52 and supplies the operation signal to the imaging device 53 and memory 54 for storing the image taken by the imaging device. Power is supplied to the imaging device 53 and the memory 54 from the main unit 10 via the connector 52.

A control unit 60 is provided within the attachment unit 50 (or, optionally, on the side of the main unit 10), and it controls overall photography operation according to operations performed by the examiner using the shutter 46 and other components.

The control unit 60 controls the turning-on or off and the amount of light of the light sources (the light source unit 100, visible light LEDs 71, 72 described hereafter, and so forth), as well as the inputting and outputting of images between the imaging device 53 and the memory 54 and the inputting and outputting of images between the CCD 40 and the monitor 41. In the present embodiment, the control unit 60 includes a pupil diameter-computing unit in which the image of the subject eye 1 taken by the CCD 40 during, for example, an alignment period is processed and recognized to compute the pupil diameter of the subject eye 1.

In the optical system shown in FIG. 3, the position conjugate with the fundus 1a of the subject eye 1 is shown at R and the position conjugate with the anterior ocular segment (especially the pupil) at P. The field stop 35 is disposed at a fundus-conjugate position with respect to the optical system (first optical system) constituted by the objective 22 and the imaging lens 33, so that the fundus image taken by the optical system is formed near the field stop. Moreover, the imaging plane of the imaging device 40 is disposed at a position conjugate with the field stop 35 with respect to the imaging lens 37, and the imaging plane of the imaging device 53 is disposed at a position conjugate with the field stop 35 with respect to the magnification lenses 47a, 47b (second optical system), so that the fundus image at the field stop 35 is re-formed by the imaging lens 37 and the magnification lenses 47a, 47b, thus allowing the fundus image to be photographed by the imaging devices 40, 53.

In the present embodiment, visible light LEDs 71, 72 for irradiating the fundus of the subject eye 1 with weak visible light prior to fundus autofluorescence (FAF) photography are added to the front side of the main unit 10 facing the subject eye 1.

As will be described referring to FIG. 6, the visible light LEDs 103 of the light source unit 100 are tuned on at a weak level of light simultaneously with the infrared light illumination by the LEDs 102 of the light source unit 100 during the subject eye observation period to illuminate the fundus of the subject eye 1 with weak visible light and promote bleaching of the visual pigment of the fundus. It is also possible to control the visible light LEDs 71, 72 in a manner similar to the visible light LEDs 103, thereby promoting bleaching of the visual pigment of the fundus and allowing a brighter fundus image to be obtained.

With such a configuration described above, the infrared light LEDs 102 are selected during the alignment and subject eye observation periods to illuminate the fundus by infrared light, and a fundus image is formed at the position of the field stop 35 by the objective 22, focus lens 32, and imaging lens 33. The fundus image at the field stop 35 passes through the infrared-passing visible light-reflecting mirror 36 and is re-formed on the imaging plane of the imaging device 40 by the imaging lens 37. This causes the fundus image to be displayed in black and white on the monitor 41 and allows the examiner to observe the fundus image on the monitor 41.

The illumination optical system is also provided with a focus dot light source 30. A light beam from the light source 30 passes through the half mirror 19 and is made incident on the fundus 1a, and the position of the focus dot changes in response to the movement of the focus lens 32, allowing the examiner to focus on the subject eye by observing the position of the focus dot. During the initial stage of alignment, an anterior ocular segment lens 42 is inserted, allowing the examiner to confirm the image of the anterior ocular segment 1b of the subject eye 1 on the monitor 41. An internal fixation lamp 43 is tuned on during alignment and focusing operations, allowing the examiner to reliably perform alignment and focusing by having the subject gaze at the fixation lamp.

Once alignment is complete, a shutter switch 46 is operated to input a shutter operation signal into the imaging device 53 and the memory 54 of the attachment unit 50 via the connector 52 and trigger the imaging device 53, initiating a process of taking a still image of the fundus. Synchronously with the operation signal from the shutter switch 46, a signal (light amount control signal) instructing the strobe light 14 to flash is sent from the imaging device 53, causing the strobe light 14 to flash. The fundus image illuminated by the flashing of the strobe light 14 is once formed at the position of the field stop 35, and again on the imaging plane of the imaging device 53 by the magnification lens 47a (47b), causing the imaging device 53 to take a still image of the fundus.

When performing fundus autofluorescence (FAF) photography, the exciter filter 13 and the barrier filter BF3 are inserted into their respective optical paths, although the detailed photography control process will be described hereafter via the embodiment shown in FIG. 6.

The still image taken by the imaging device 53 is stored in the memory 54 in the attachment unit 50. The still image stored in the memory 54 is inputted into an external PC (not shown), displayed on a monitor 41, or outputted to a printer (not shown). Alternatively, the memory 54 may be configured like a cartridge that is capable of being removed from the attachment unit 50 and inserted to anther device to read out memory contents in the device.

The fundus image can taken with photography magnification varied by the magnification lenses 47a, 47b disposed in the photography optical system or using zoom lenses substituted therefor. At high levels of magnification, a fundus image is taken so large as the field stop 35 is not photographed, and, at low levels of magnification, the field stop is also photographed together with the fundus image.

FIG. 6 shows an example of fundus autofluorescence (FAF) photography control according to the present invention, and primarily shows a fundus autofluorescence (FAF) photography operation performed using the configuration shown in FIG. 3.

In step S1 in FIG. 6, the examiner manipulates a joystick of the apparatus to move the main unit 10 toward the subject eye 1 and performs alignment (rough alignment) using the image of the anterior ocular segment 1b. At this time, the infrared light LEDs 102 of the light source unit 100 are turned on, and the anterior ocular segment lens 42 is inserted into the optical path. This causes the anterior ocular segment 1b of the subject eye 1 to be irradiated with infrared light, an image of the anterior ocular segment 1b to be formed on the imaging device 40 by the light reflected from the anterior ocular segment 1b, and its image to be displayed on a screen of the monitor 41. The examiner manipulates the XY stage while looking at the display to align the main unit 10.

When a pupil center 1c in the image of the anterior ocular segment 1b is aligned with the center of a marker (not shown) on the screen of the monitor 41, as shown to the right of step S2, the examiner completes anterior ocular segment alignment (step S2).

An operation unit not shown in the drawings is used to perform a predetermined operation indicating the end of alignment and the beginning of photography. The visible light LEDs 103 of the light source unit 100 (or visible light LEDs 71, 72 on the front side of the main unit) are turned on to irradiate the fundus of the subject eye E with weak visible light prior to fundus autofluorescence (FAF) photography (step S3). The light amount of the visible light LEDs 103 of the light source unit 100 (or LEDs 71, 72) is gradually increased with every pass through step S3.

In step S4, an image of the anterior ocular segment is imported to the pupil diameter-computing unit in the control unit 60 to measure and compute the pupil diameter of the subject eye 1 (step S41). Positional information for the focus lens 32 is also referred to during this computation.

Turning on the visible light LEDs 103 of the light source unit 100 (or visible light LEDs 71, 72) promotes the bleaching of the visual pigment of the fundus, and enhances fundus autofluorescence. However, photography will be impossible when large pupillary contraction in the subject eye 1 occurs, so that the light amount of the visible light LEDs 103 of the light source unit 100 (or visible light LEDs 71, 72) is controlled within a photographable range in a loop constituted by steps S3, S4, S41, and S42.

It is confirmed whether the pupil diameter of the subject eye 1 calculated by the pupil diameter-computing unit has reached a minimum photographable pupil diameter (step S42), and, if it is positive, the light amount of the visible light LEDs 103 of the light source unit 100 (or visible light LEDs 71, 72) is fixed (step S43), and the procedure moves to step S5. If there is still room before the pupil diameter of the subject eye 1 calculated by the pupil diameter-computing unit reaches the minimum photographable pupil diameter, the process returns from step S42 to step S3 and the light amount of the visible light LEDs 103 of the light source unit 100 (or visible light LEDs 71, 72) is gradually increased.

The anterior ocular segment lens 42 is removed from the optical path immediately after the anterior ocular segment image is imported (step S5). This causes the image of the fundus 1a to be formed on the imaging device 40 and displayed on the screen of the monitor 41.

The examiner performs fine alignment with respect to the fundus by manipulating the joystick while looking at the fundus image, and adjusts focus according to the diopter of the subject eye 1 while moving the focus lens 32 and observing a dot m on the monitor 41 (step S6).

Next, the examiner drives the XY stage while observing the fundus image and moves the main unit 10 so that a desired measurement region on the fundus image reaches a predetermined position in the photographic field of view (step S7), and, once measurement region positioning is complete (step S8), the examiner turns the shutter switch 46 on (step S9). After the shutter opens in response, the strobe light 14 flashes (step S11).

Before this, data for the fundus image from the imaging device 40 is imported to a recording unit (step S10) to record the fundus image data and the fundus measurement region position data in the memory 54.

If lipofuscin is accumulated in the retina of the fundus 1a, the flashing of the strobe light 14 excites the lipofuscin, generating natural fluorescence. At this time, the fundus of the subject eye 1 is previously irradiated with visible light by the visible light LEDs 103 of the light source unit 100 (or LEDs 71, 72), thereby promoting visual pigment bleaching and enhancing fundus autofluorescence and allowing a brighter FAF image to be taken. The barrier filter BF3 is disposed in the light-receiving optical system, allowing only natural fluorescence from the measurement region on the fundus 1a to be incident on the CCD 53. A fundus image of the natural fluorescence is electronically taken by the CCD 53 and recorded in the memory 54 (step S12).

As described above, the adjustment means is provided that alters the ratio of the visible light component to the infrared light component of the illumination light source, allowing the fundus of the subject eye E to be irradiated with weak visible light during the subject eye observation period prior to the fundus autofluorescence (FAF) photography and the light amount to be maximally selected and fixed within a range not impeding photography. This promotes the bleaching of the visual pigment of the fundus of the subject eye, enhancing fundus autofluorescence and allowing a bright FAF image to be taken.

### Key to the Symbols

- LA, LA': Lamp
- L1, L1': Condenser lens
- M2: Reflection mirror
- L3, L4: Relay lens
- M4, M5,: M6 Return mirror
- L5: Objective
- L6: Focus lens
- L7: Imaging lens
- L8: Eyepiece
- L9, L11: Lens
- L20: Lens
- 117: Working dot light source
- 121: Switching operation means
- 122: Switching unit
- 123: Display unit
- 124: Recording device
- 125: LAN
- 126: Personal computer (PC)
- 130: Timer
- 1: Subject eye
- 3: Barrier filter BF
- 10: Main unit
- 13: Exciter filter
- 14: Strobe light
- 15: Diffuser plate
- 16: Ring slit
- 17: Lens
- 18: Black-spot plate
- 19: Half mirror
- 20: Relay lens
- 21: Perforated reflection mirror
- 22: Objective
- 30: Focus dot light source
- 31: Photography aperture
- 32: Focus lens
- 33: Imaging lens
- 34: Half mirror
- 35: Field stop
- 36: Mirror
- 37: Imaging lens
- 40: CCD
- 41: Monitor
- 42: Anterior ocular segment lens
- 43: Internal fixation lamp
- 46: Shutter
- 47a: Magnification lens
- 50: Attachment unit
- 51: Mount
- 52: Connector
- 53: Imaging device
- 54: Memory
- 60: Control unit
- 71, 72: LED
- 100: Light source unit
- 101: Board
- 102, 103: LED

## Claims

1. An ophthalmologic photography apparatus comprising:
an illumination optical system for illuminating the fundus of a subject eye using illumination light from an illumination light source;
a photography optical system for taking a fundus image of the fundus irradiated with the illumination light by the illumination optical system;
a first electronic infrared imaging means for taking a video for observing the fundus of the subject eye during a subject eye observation period prior to taking a still image of the subject eye; and
a second electronic imaging means for taking a still image of the subject eye;
wherein the subject eye observation period in which the fundus of the subject eye is observed using the first electronic imaging means is followed by taking a still image of the subject eye using the second imaging means in response to a shutter operation and recording the obtained still image as still image information;
the ophthalmologic photography apparatus being provided with:
an exciter filter removably provided in an optical path of the illumination optical system for photographing natural fluorescence emitted by the fundus of the subject eye;
a barrier filter removably provided in an optical path of the photography optical system for photographing natural fluorescence emitted by the fundus of the subject eye; and
an adjustment means for varying the ratio of the visible light component to the infrared light component of the illumination light.

2. An ophthalmologic photography apparatus according to claim 1, wherein the adjustment means is capable of independently adjusting the amount of light of visible light component and infrared light component of the illumination light from the illumination light source.

3. An ophthalmologic photography apparatus according to claim 2, wherein, in a natural fluorescence photography mode, when the exciter filter for photographing the natural fluorescence of the fundus of the subject eye is inserted into the optical path of the illumination optical system, adjustment of the amount of light from the visible illumination light source by the adjustment means is only possible during anterior ocular segment alignment

4. An ophthalmologic photography apparatus according to claim 3, wherein, in a natural fluorescence photography mode, when the exciter filter for photographing the natural fluorescence of the fundus of the subject eye is inserted into the optical path of the illumination optical system, the amount of the visible light component of the illumination light from the illumination light source is gradually increased using the adjustment means once anterior ocular segment alignment with respect to the subject eye has been attained, and, when the pupil diameter of the subject eye is detected to have reached a predetermined level of contraction, the amount of the visible light component of the illumination light from the illumination light source is fixed for transition to a fundus alignment mode.
